Europäisches Patentamt

European Patent Office

Office européen des brevets

(11) Publication number: **0 099 758**
**B1**

## (12) EUROPEAN PATENT SPECIFICATION

(45) Date of publication of patent specification: **12.10.88**

(21) Application number: **83304203.9**

(22) Date of filing: **20.07.83**

(51) Int. Cl.⁴: **A 61 F 13/00, A 61 L 15/00**

(54) **Composite wound dressing.**

(30) Priority: **21.07.82 GB 8221044**

(43) Date of publication of application:
**01.02.84 Bulletin 84/05**

(45) Publication of the grant of the patent:
**12.10.88 Bulletin 88/41**

(84) Designated Contracting States:
**DE FR GB IT**

(56) References cited:
**US-A-3 903 882**
**US-A-4 300 575**

**LIST & HORHAMMER "Hagers Handbuch der Pharmazeutischen Praxis" 4th Edition, vol. 7, part A: "Arzneiformen", 1971 Springer Verlag (DE)**

(73) Proprietor: **University of Strathclyde McCance Building 16 Richmond Street Glasgow G1 1YQ, Scotland (GB)**

(72) Inventor: **Juhasz, Laszlo, Dr. Kilmardinny Avenue Bearsden Glasgow Scotland (GB)**

(74) Representative: **Wotherspoon, Graham et al FITZPATRICKS 4 West Regent Street Glasgow G2 1RS Scotland (GB)**

Courier Press, Leamington Spa, England.

EP 0 099 758 B1

## Description

The present invention relates to composite wound dressings and particularly, but not exclusively, for use as a general surgical wound dressing, burn dressing, donor site dressing, bedsore dressing, infected ulcer dressing and like applications. It may also be used as an electrode dressing for transcutaneous pain relief or functional stimulation.

In order for a composite wound dressing to be as effective and efficient as normal skin, it must stimulate the function of normal skin. There are three main requirements that normal skin fulfils; firstly it provides controlled water vapour permeability, secondly the skin provides a tough mechanical barrier between the tissues and the external environment with uniform pressure distribution and acts as a barrier to infection and thirdly, it provides a bioactive tissue interface to maintain an adaptive physiological performance and intracellular/extracellular activities in wound repair. It is therefore considered that a composite wound dressing should satisfy all of these basic requirements and take part in the fundamental activities of the wound healing process. The author considers that at the present time, there is no composite wound dressing available which satisfies all these requirements.

US—A—3 903 882 discloses a flexible, tissue-surface-conformable, water vapour-permeable, composite dressing for wounds, particularly burns, formed from a knitted fabric of a tissue absorbable polymer, eg polyglycolic acid, having on the non-tissue-contacting surface a water vapour-permeable elastomeric film, eg polyurethane, silicone, polycaprolactone and natural rubber, to provide an essentially two-layered dressing. US—A—4 300 575 discloses a disposable electrode which is not intended for use with open wounds. Although air-permeable, it cannot be successfully applied to open wounds because the skin engageable surface is formed from an open cell structure which is liable to adhere to the open wound. Further, the conductive particles used therein may be shed into an open wound leading to undesirable granuloma formation, for example.

Subject of the European patent application 86116467.1, published on 08.07.87 as EP—A—227955 is a composite wound dressing comprising in a layered arrangement, a semipermeable membrane and a non-stick, self-sealing biodegradable tissue interface, characterised in that said semipermeable membrane is formed from a synthetic collagen, an alginate or a biocompatible polymer selected from polyurethane, polypropylene and silicone rubber, and in that the biodegradable tissue interface is formed from a synthetic collagen, a Na/Ca alginate or a collagen/alginate complex.

In this composite wound dressing a supporting and reinforcing permeable layer may be located ·between the semipermeable membrane and the biodegradable tissue interface.

It is an object of the present invention to obviate or to mitigate the disadvantages associated with current composite wound dressings.

According to the present invention there is provided a composite wound dressing comprising in a layered arrangement, a semi-permeable membrane and a non-stick, self-sealing biodegradable tissue interface, characterised in that said dressing further comprises a permeable reinforcing layer located between the semi-permeable membrane and the biodegradable tissue interface, which layer is formed from an electrically-conductive material.

Preferably, said permeable layer is polyester, polyethylene or cotton fabric coated with carbon doped silicon or natural rubber. Alternatively, said fabrics are plated or deposited with metals such as silver, zinc, gold, platinum and the like.

Alternatively, said permeable layer is charcoal cloth fabric consisting of substantially 100% activated carbon, said cloth fabric being produced by carbonising and activating (700—1200)°C a woven viscose rayon fabric.

Preferably also, said biodegradable interface is a synthetic collagen produced from animal sources such as calf skin and intestines. Alternatively, the biodegradable tissue interface is a Na—Ca alginate produced from seaweeds. Preferably also, the said biodegradable tissue interface is a collagen-alginate complex with ratio of (0.1—30) and it is prepared as porous structure.

Preferably also, the semipermeable membrane is a synthetic collagen. Alternatively, the semipermeable membrane may be an alginate or a biocompatible polymer such as polyurethane, polypropylene, silicone rubber, etc.

Embodiments of the present invention will now be described by way of example with reference to the accompanying drawings in which:—

Fig. 1 is a schematic diagram of a composite wound dressing in accordance with the present invention; and

Fig. 2 is an alternative diagram of another composite wound dressing in accordance with the present invention;

Fig. 3 is a schematic diagram of a composite wound dressing for use on burns in accordance with the present invention.

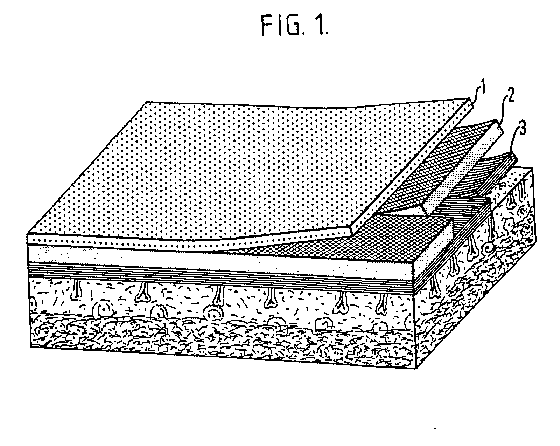

Referring now to Fig. 1 of the drawings, the composite wound dressing comprises: a calcium alginate semipermeable membrane 1, an electrically conductive silverester fabric 2, and a biodegradable Na—Ca alginate (BdA) 3.

The biodegradable alginate is produced from brown seaweed (phaeophycae) which contains naturally occurring polysaccharides. The BdA is prepared by purifying and acidifying to a various degree the sodium alginate to ·obtain a slow dissolving material. In principle therefore BdA contains both sodium and calcium alginate with a ratio of (Na/Ca)=0.1 to 50.

The dressing is placed on a wound, for example a burn with the biodegradable alginate in contact with the wound surface. Once the BdA 3 is in contact with tissue fluid, ie such as exudate, it dissolves at a predetermined rate. In this hydrolysed state it produces a viscous polyelectrolyte film which provides haemostatic wound sealing an electrical charge transfer of the sodium and calcium ions from an external source. The multilayered design is similar in structure to normal skin and provides near physiological performance since the semipermeable membrane controls the rate of water vapour transmission from the tissue. The supporting fabric provides increased mechanical strength with uniform pressure and electrical field distribution and the biodegradable tissue interface 3 provides haemostatic sealing and influences when electrically energised intracellular and extracellular activities in the wound repair process.

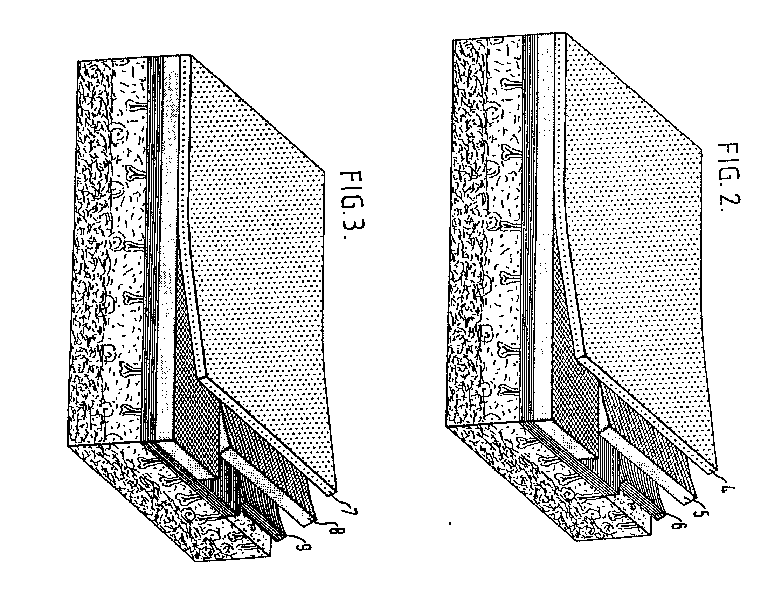

With regard to the composite wound dressing as shown in Fig. 2, a polyurethane polymer 4 is the semipermeable membrane, the supporting fabric 5 is a cotton fabric coated with carbon-doped silicon or natural rubber, and the biodegradable tissue interface 6 is a synthetic collagen produced from calf skin or calf intestine or from other animal sources. After several stages of purification a collagen film or fabric is prepared and used. This composite wound dressing functions in a similar way to that described with reference to Fig. 1, however, the carbon-doped silicone rubber is the electrically conductive reinforcing permeable layer.

An external electro-motive-force or magnetic field (both denoted as EMF) can influence both normal neuro-vascular processes and tissue repair mechanisms. The doping should not give preferably electrical resistance greater than 600 ohms/square unit since this necessitates providing a higher voltage to maintain a current within the tissue sufficient to provide EMF assisted wound healing. A typical example for current density is (1—10) $\mu A/cm^2$.

With respect to neurovascular control, an externally applied EMF will induce localised vasoconstriction to re-establish the balanced osmotic feedback between the blood vessels and the neighbouring tissue. Thus, the widened and damaged capillary bed, due to trauma induced histamine stimulation, will contract due to the applied EMF.

The protein concentration in the blood vessels will be maintained at a high level and consequently water will be drawn back into these vessels by osmosis. This re-establishment of osmotic feedback prevents the accumulation of both water and proteins in the surrounding tissue, thus reducing local pain and inflammation due to the fluid pressure on the sensory nerve endings.

Additionally, an externally applied EMF can accelerate the tissue regeneration aspect of healing by restoring order to the basic biological processes of all division and synthesis which give rise to the large numbers of phagocytes necessary for epithelialisation to proceed. Here the postulated mechanism is one of stabilisation of the normal cellular activities followed by an early acceleration of the cell synthesis required for tissue reconstruction.

Results showed that wounds treated with such a dressing and having external EMF's applied, healed better and quicker than unstimulated wounds with minimum scar formation.

With regard to the wound dressing shown in Fig. 3 a polyurethane polymer 7 is the semipermeable membrane, the supporting fabric is a charcoal cloth fabric 8 and a synthetic collagen-alginate 9 is the biodegradable tissue interface. This composite wound dressing can be connected to a source of electrical energy and is particularly suitable for treating burn wounds.

In addition, topical growth promoting, antibacterial or antiallergic agents such as silver sulphadiazene, zinc and other substrances may be incorporated into the dressing, preferably into the collagen, alginate or collagen-alginate of the biodegradable tissue interface.

The advantages of the composite wound dressing according to the present invention include; control of the rate of water vapour transmission from the wound thereby presenting local dehydration, all processes in the wound healing phase are enhanced, eg inflammation is reduced, epithelialisation and collagen synthesis is increased, its layered structure gives it flexibility and mechanical strength and facilitates easy handling, the electrical conductivity of the supporting layer can be used to facilitate postoperative pain relief, and the dressing is self-sealing non-tissue adhesive, simply peeling off when required, when the dressing is used in the EMF assisted mode, the collagen synthesis phase of the wound healing is greatly enhanced thereby increasing the strength of the wound in the long term, and reduced scar formation and improved clinical appearance are an advantage of using this composite wound dressing.

**Claims**

1. A composite wound dressing comprising in a layered arrangement, a semi-permeable membrane and a non-stick, self-sealing biodegradable tissue interface, characterised in that said dressing further comprises a permeable reinforcing layer located between the semi-permeable membrane and the biodegradable tissue interface, which layer is formed from an electrically-conductive material.

2. A composite wound dressing according to claim 1 wherein the permeable layer is a polyester, polyethylene, rayon, or cotton fabric coated with carbon-doped silicone or natural rubber.

3. A composite wound dressing according to claim 1 wherein the permeable layer is a polyester, polyethylene, rayon or cotton fabric plated or deposited with silver, zinc, gold, platinum or carbon.

4. A composite wound dressing according to claim 1 wherein the permeable layer is a charcoal cloth fabric.

5. A composite wound dressing according to claim 4 wherein the said cloth fabric is produced by carbonising and activating a woven viscose rayon fabric at a temperature in the range of from 700° to 1200°C.

6. A composite wound dressing according to any one of the preceding claims wherein the semi-permeable membrane is formed from a synthetic collagen, alginate, polyurethane, polypropylene, silicone or natural rubber.

7. A composite wound dressing according to any one of the preceding claims wherein the tissue interface is formed from a biodegradable alginate and/or collagen.

8. A composite wound dressing according to claim 7 wherein the biodegradable alginate is a sodium-calcium alginate mixture in which the ratio of sodium to calcium is 0.1:50.

9. A composite wound dressing according to claim 7 wherein the tissue interface is formed from a collagen-alginate complex in which the ratio of collagen to alginate is 0.1:30.

## Patentansprüche

1. Schichtförmiger Wundverband, der in einer lagenförmigen Anordnung eine semi-permeable Membran sowie eine nichtklebende, selbstverschließende und biologisch abbaubare Gewebeauflage aufweist, dadurch gekennzeichnet, daß der Wundverband außerdem eine durchlässige verstärkende Schicht aufweist, die zwischen der semi-permeablen Membran und der biologisch abbaubaren Gewebeauflage angeordnet ist, wobei diese Schicht aus elektrisch leitendem Material gebildet ist.

2. Schichtförmiger Wundverband nach Anspruch 1, bei dem die durchlässige Schicht eine Polyester-, Polyäthylen-, Rayon- oder aus Baumwollware ist, die mit mit Kohlenstoff dotiertem Silikon oder Naturgummi beschichtet ist.

3. Schichtförmiger Wundverband nach Anspruch 1, dadurch gekennzeichnet, daß die durchlässige Schicht eine Polyester-, Polyäthylen-, Rayon- oder Baumwollware ist, die mit Silber, Zink, Gold, Platin oder Kohlenstoff überzogen oder auf der Silber, Zink, Gold, Platin oder Kohlenstoff abgelagert ist.

4. Schichtförmiger Wundverband nach Anspruch 1, bei dem die durchlässige Schicht ein kohlenhaltiger Textilstoff ist.

5. Schichtförmiger Wundverband nach Anspruch 4, dadurch gekennzeichnet, daß der Textilstoff durch Verkohlen und Aktivieren einer Viskose-Rayon-Webware bei einer Temperatur im Bereich von 700° bis 1200°C hergestellt ist.

6. Schichtförmiger Wundverband nach einem der vorhergehenden Ansprüche, dadurch gekennzeichnet, daß die semi-permeable Membran aus synthetischem Kollagen, Alginat, Polyurethan, Polypropylen, Silikon oder Naturgummi gebildet ist.

7. Schichtförmiger Wundverband nach einem der vorhergehenden Ansprüche, dadurch gekennzeichnet, daß die Gewebeauflage aus einem biologisch abbaubaren Alginat und/oder Kollagen gebildet ist.

8. Schichtförmiger Wundverband nach Anspruch 7, dadurch gekennzeichnet, daß das biologisch abbaubare Alginat ein Natrium-Kalzium-Alginat-Gemisch ist, bei dem das Verhältnis von Natrium zu Kalzium 0,1:50 ist.

9. Schichtförmiger Wundverband nach Anspruch 7, dadurch gekennzeichnet, daß die Gewebeauflage aus einem Kollagen-Alginat-Komplex gebildet ist, bei dem das Verhältnis von Kollagen zu Alginat 0,1:30 ist.

## Revendications

1. Pansement composite pour blessure comprenant, dans un agencement en couches, une membrane semi-perméable et une interface côté tissus non collante, à auto-fermeture et biodégradable, caractérisé en ce que ledit pansement comprend en outre une couche perméable de renforcement placée entre la membrane semi-perméable et l'interface biodégradable côté tissus, cette couche étant constituée d'une matière électriquement conductrice.

2. Pansement composite pour blessure suivant la revendication 1, dans lequel la couche perméable est une étoffe de polyester, polyéthylène, rayonne ou coton, revêtue de silicone ou caoutchouc naturel dopé au carbone.

3. Pansement composite pour blessure suivant la revendication 1, dans lequel la couche perméable est une étoffe de polyester, polyéthylène, rayonne ou coton, plaquée ou revêtue d'argent, zinc, or, platine ou carbone.

4. Pansement composite pour blessure suivant la revendication 1, dans lequel la couche perméable est une étoffe de charbon.

5. Pansement composite pour blessure suivant la revendication 4, dans lequel ladite étoffe est produite par carbonisation et activation d'une étoffe tissée de rayonne de viscose, à une température comprise entre 700°C et 1200°C.

6. Pansement composite pour blessure suivant l'une quelconque des revendications précédentes, dans lequel la membrane semi-perméable est formée à partir de collagène synthétique, alginate, polyuréthane, polypropylène, silicone ou caoutchouc naturel.

7. Pansement composite pour blessure suivant l'une quelconque des revendications précédentes, dans lequel l'interface avec les tissues est formée à partir d'alginate et/ou de collagène biodégradable.

8. Pansement composite pour blessure suivant la revendication 7, dans lequel l'aginate biodégradable est un alginate de sodium-calcium dans lequel le rapport du sodium au calcium est de 0,1/50.

9. Pansement composite pour blessure suivant la revendication 7, dans lequel l'interface côté tissus est formée d'un complexe de collagène-alginate dans lequel le rapport du collagène à l'alginate est de 0,1/30.

FIG. 1.

FIG. 2.

4

5

6

FIG. 3.

7

8

9